# FASCICULE DE BREVET EUROPEEN

(11) **EP 1 492 754 B1**
(45) Date de publication et mention de la délivrance du brevet: **05.11.2008**
(21) Numéro de dépôt: 03745809.8
(22) Date de dépôt: 28.03.2003
(51) Int. Cl.: C07C 51/23, C07C 55/14, C07C 53/00, C07C 55/21, C07C 57/30

(54) **PROCEDE DE FABRICATION D'ACIDES CARBOXYLIQUES**
VERFAHREN ZUR HERSTELLUNG VON CARBONSÄUREN
METHOD FOR MAKING CARBOXYLIC ACIDS

(30) Priorité: 08.04.2002 FR 0204332
(43) Date de publication de la demande: 05.01.2005
(73) Titulaire: RHODIA POLYAMIDE INTERMEDIATES, 69190 Saint-Fons (FR)
(72) Inventeur: BONNET, Didier, F-69004 LYON (FR); FACHE, Eric, F-69300 CALUIRE (FR); SIMONATO, Jean-Pierre, F-38360 SASSENAGE (FR); VERACINI, Serge, F-69005 LYON (FR)
(74) Mandataire: Esson, Jean-Pierre
(86) Numéro de dépôt international: PCT/FR2003/000984
(87) Numéro de publication internationale: WO 2003/084913

(56) Documents cités:
- EP-A- 0 519 569
- EP-A- 0 784 045
- DE-A- 19 941 315
- US-A- 3 726 917
- US-A- 5 321 157
- DATABASE WPI Section Ch, Week 197705 Derwent Publications Ltd., London, GB; Class E17, AN 1977-08696Y XP002217021 & JP 52 000931 B (TEIJIN LTD), 11 janvier 1977 (1977-01-11)

## Description

La présente invention concerne un procédé de fabrication d'acides carboxyliques par oxydation d'un composé hydroperoxyde d'alkyle.

Elle se rapporte plus particulièrement à l'oxydation d'hydroperoxyde de cyclohexyle en acide adipique par un agent oxydant contenant de l'oxygène moléculaire.

La fabrication d'acide adipique par oxydation du cyclohexane est un procédé qui a été étudié depuis de nombreuses années. En effet, l'acide adipique est un composé chimique important utilisé comme matière première dans de nombreuses fabrications telles que la production de polymères comme les polyamides, polyesters ou polyuréthannes.

Plusieurs procédés de fabrication d'acide adipique à partir d'hydrocarbures tels que benzène, phénol, cyclohexène, cyclohexane ont été proposés.

L'oxydation du cyclohexane soit directement soit en deux étapes sont les voies les plus étudiées pour produire l'acide adipique.

Ainsi, à titre d'illustration des procédés d'oxydation directe des hydrocarbures en acides dicarboxyliques, le brevet américain 2,223,493 publié en décembre 1940, décrit l'oxydation d'hydrocarbures cycliques en diacides correspondants, en phase liquide comportant généralement de l'acide acétique, à une température d'au moins 60°C, à l'aide d'un gaz contenant de l'oxygène et en présence d'un catalyseur d'oxydation tel qu'un composé du cobalt.

De nombreux autres brevets et articles décrivent cette réaction d'oxydation directe du cyclohexane en acide adipique. Toutefois, pour obtenir des rendements acceptables de production d'acide adipique, ces documents décrivent l'utilisation de l'acide acétique comme solvant, en présence soit de catalyseur homogène soit de catalyseur hétérogène. On peut citer, à titre d'illustration, l'article paru dans le journal "Chemtech", 555-559 (septembre 1974) dont l'auteur est K. Tanaka qui résume et commente le procédé d'oxydation directe du cyclohexane. On peut également citer les brevets américains 3,231,608 ; 4,032,569 ; 4,158,739; 4,263,453 et 5,321,157, le brevet européen 870751 qui décrivent différents systèmes catalytiques homogènes.

Il a également été proposé des procédés d'oxydation directe du cyclohexane en présence de catalyseur hétérogène comme des aluminophosphates substitués par du cobalt, comme dans le brevet européen n°519569.

Il a également été proposé quelques procédés d'oxydation en une seule étape du cyclohexane en acide adipique sans utilisation d'acide acétique. Certains proposent de réaliser cette réaction en l'absence de solvants, d'autres avec des solvants tels que des esters organiques comme les acétates (US 4,098,817), de l'acétone (US 2,589,648) ou encore des alcools comme le butanol, le méthanol, le cyclohexanol ou l'acétonitrile (Ep 784045). Enfin, il a été proposé par la demande de brevet WO 0166502, un procédé d'oxydation directe d'hydrocarbures en acides dicarboxyliques en présence d'un acide carboxylique à caractère lipophile comme solvant. Ce solvant permet de remédier aux inconvénients présentés par l'acide adipique.

Ces procédés conduisent généralement à des sélectivités en acide adipique très faibles. Par ailleurs, les solvants utilisés présentent souvent une faible stabilité dans les conditions d'oxydation de l'hydrocarbure comme le cyclohexane. Cette faible stabilité provoque une consommation importante du solvant ce qui rend difficile l'exploitation industrielle de tels procédés.

Les procédés de fabrication d'oxydation d'hydrocarbures en acides dicarboxyliques par deux étapes successives d'oxydation sont utilisés industriellement à grande échelle. Ces procédés consistent, dans une première étape, à réaliser l'oxydation des hydrocarbures en alcools et cétones par l'oxygène ou un gaz contenant de l'oxygène. Dans une seconde étape, les alcools et/ou cétones sont oxydés en acides par une oxydation nitrique. Différents modes de réalisation de ces deux étapes sont exploités. Ainsi, la première étape peut comprendre deux sous-étapes, dans une première sous-étape l'hydrocarbure est oxydé en hydroperoxyde. Après séparation de l'hydroperoxyde de l'hydrocarbure qui n'a pas réagi, l'hydroperoxyde est décomposé dans un réacteur distinct en alcool et/ou cétone. Dans un autre mode de réalisation également exploité, la production d'hydroperoxyde et sa décomposition en alcool et/ou cétone sont réalisées simultanément dans un seul réacteur.

Actuellement, l'oxydation des alcools et/ou cétones est réalisée par l'acide nitrique. Une telle réaction produit comme effluent principal des oxydes nitreux et nitriques imposant la présence d'un procédé de traitement de ceux-ci pour diminuer l'effet sur l'environnement. Ce traitement des effluents pénalise économiquement les procédés d'oxydation en deux étapes.

Le brevet US 3361806 décrit un procédé d'oxydation d'un mélange de cyclohexanone, cyclohexanol et produits oxygénés.

Un des buts de la présente invention est de proposer un procédé d'oxydation d'hydrocarbures pour produire des acides ou polyacides, ne requérant pas l'utilisation comme agent d'oxydation d'acide nitrique ou un de ses dérivés et donc ne produisant pas de rejet d'oxydes d'azote.

A cet effet, l'invention propose un procédé de fabrication d'acides carboxyliques caractérisé en ce qu'il consiste à faire réagir un hydroperoxyde d'hydrocarbures avec de l'oxygène ou un gaz contenant de l'oxygène en présence d'un catalyseur d'oxydation comprenant un métal appartenant aux groupes des métaux de transition.

Le catalyseur peut comprendre avantageusement un élément métallique choisi dans le groupe comprenant Cu, Ag, Au, Mg, Ca, Sr, Ba, Zn, Cd, Hg, Al, Sc, In, TI, Y, Ga, Ti, Zr, Hf, Ge, Sn, Pb, V, Nb, Ta, Cr, Mo, W, Mn, Tc, Re, Fe, Ru, Os, Co, Rh, Ir, Ni, Pd, Pt, les lanthanides comme Ce et les combinaisons de ceux-ci.

Ces éléments catalytiques sont mis en oeuvre soit sous forme de composés avantageusement au moins partiellement solubles dans le milieu liquide d'oxydation aux conditions de mise en oeuvre de la réaction d'oxydation, soit supportés, absorbés ou liés à un support inerte tel que silice, alumine, par exemple.

Dans le cas d'une catalyse hétérogène, les éléments métalliques catalytiquement actifs sont supportés ou incorporés dans une matrice minérale micro ou mésoporeuse ou dans une matrice polymérique ou sont sous forme de complexes organométalliques greffés sur un support organique ou minéral. Par incorporé, on entend que le métal est un élément du support ou que l'on travaille avec des complexes stériquement piégés dans des structures poreuses dans les conditions de l'oxydation.

Dans un mode de réalisation préféré de l'invention, le catalyseur homogène ou hétérogène est constitué de sels ou de complexes de métaux des groupes IVb (groupe du Ti), Vb (groupe du V), Vlb(groupe du Cr), Vllb (groupe du Mn), VIII (groupe du Fe ou Co ou Ni), Ib (groupe du Cu), et cérium, seuls ou en mélange. Les éléments préférés sont, en particulier, Co et/ou Mn et/ou Cr et/ou Zr, Hf, Ce et/ou Zr, Hf. La concentration en métal dans le milieu liquide d'oxydation varie entre 0,00001 et 5 % (% poids), de préférence entre 0,0001% et 2%.

Selon l'invention, les hydroperoxydes qui sont mis en oeuvre dans le procédé de l'invention sont de manière générale les hydroperoxydes primaires ou secondaires dérivant des alcanes, des cycloalcanes, des hydrocarbures alkyl-aromatiques dont le cycle aromatique comporte éventuellement un ou plusieurs substituants tels que notamment groupe alkyle ou atome d'halogène plus particulièrement atome de chlore, des alcènes et des cycloalcènes ayant de 3 à 20 atomes de carbone.

A titre d'exemples de tels hydroperoxydes, on peut citer l'hydroperoxyde de cyclohexyle, l'hydroperoxyde de cyclododécyle, l'hydroperoxyde de la tétraline, l'hydroperoxyde d'éthylbenzène, l'hydroperoxyde du pinane.

Parmi ces hydroperoxydes, l'un des plus intéressants est très certainement l'hydroperoxyde de cyclohexyle dont l'oxydation conduit à l'acide adipique comme acide dicarboxylique prépondérant, l'un des composés de base pour la fabrication de polyamides, plus particulièrement du polyhexaméthylèneadipate.

Ces hydroperoxydes peuvent être obtenus par différents procédés et utilisés dans le procédé de l'invention sous forme purifiée ou en mélange avec d'autres composés provenant notamment de leurs procédés de fabrication.

Le procédé de l'invention peut être mis en oeuvre de préférence en présence d'un solvant constitué avantageusement par l'hydrocarbure utilisé pour la fabrication de l'hydroperoxyde. Toutefois, on peut faire appel à divers solvants tels que les alcanes parmi lesquels on citera plus particulièrement l'hexane, l'heptane et l'isooctane ; les cycloalcanes parmi lesquels on mentionnera à titre illustratif le cyclohexane et le cyclooctane, les hydrocarbures aromatiques tels le benzène, le toluène et le xylène, les hydrocarbures halogénés, les alcools, les cétones, les éthers, les ntriles, les acides carboxyliques comme l'acide acétique et les mélanges de ces solvants

Toutefois il est à noter que l'hydroperoxyde étant généralement produit sous la forme d'une solution dans un hydrocarbure, par exemple le cyclohexane, par oxydation de celui-ci, la réaction d'oxydation est avantageusement réalisée sur une solution provenant de l'oxydation de l'hydrocarbure (cyclohexane). Cette solution peut être utilisée en l'état ou après élimination de certains constituants de manière en soi connue. Il est également possible d'utiliser une solution d'hydroperoxyde dans le solvant, par exemple, le cyclohexane sensiblement pur.

Ainsi, le procédé de l'invention pourra être réalisé sur une solution provenant de l'oxydation d'un hydrocarbure en hydroperoxyde telle quelle ou après élimination de certains sous-produits par, par exemple, lavage de la solution par de l'eau pour éliminer notamment les acides hydrosolubles ou sur l'hydroperoxyde purifié par les procédés classiques de purification tels que distillation, extraction ou tout autre méthode classique.

La réaction d'oxydation est mise en oeuvre à une température comprise entre 50°C et 250°C, de préférence entre 70°C et 200°C. Elle peut être réalisée sous pression atmosphérique. Toutefois, elle est généralement mise en oeuvre sous pression pour maintenir les composants du milieu réactionnel sous forme liquide. La pression peut être comprise entre 10 KPa (0,1 bar) et 20000 KPa (200 bars), de préférence entre 100Kpa (1 bar) et 10000 Kpa (100 bar).

L'oxygène utilisé peut être sous forme pure ou en mélange avec un gaz inerte tel que l'azote ou l'hélium. On peut également utiliser de l'air plus ou moins enrichi en oxygène.

Le procédé d'oxydation peut être réalisé de manière continue ou selon un procédé discontinu. Avantageusement, le milieu réactionnel liquide sorti du réacteur est traité selon des procédés connus permettant d'une part de séparer et récupérer les acides produits et d'autre part recycler les composés organiques non oxydés ou partiellement oxydés comme le cyclohexane, le cyclohexanol et/ou le cyclohexanone, le catalyseur et éventuellement le solvant.

La quantité de catalyseur, exprimée en pourcentage pondéral de métal par rapport au mélange réactionnel, se situe généralement entre 0,00001 % et 5 % et de préférence entre 0,0001 % et 2 %, sans que ces valeurs soient critiques. Il s'agit cependant d'avoir une activité suffisante tout en n'utilisant pas des quantités trop importantes d'un catalyseur qu'il faut ensuite séparer du mélange réactionnel final et recycler.

Il peut être avantageux de mettre en oeuvre également un composé initiateur de la réaction d'oxydation, tel que par exemple une cétone, un aldéhyde ou un hydroperoxyde. La cyclohexanone qui est un intermédiaire réactionnel dans le cas de l'oxydation du cyclohexane, est tout particulièrement indiquée. Généralement l'initiateur représente de 0,01 % à 20 % en poids du poids du mélange réactionnel mis en oeuvre, sans que ces proportions aient une valeur critique. L'initiateur est surtout utile lors du démarrage de l'oxydation et lorsque l'on réalise l'oxydation à une température inférieure à 120°C. Il peut être introduit dès le début de la réaction.

Il est également possible, sans pour cela sortir du cadre de l'invention, d'ajouter dans le milieu réactionnel un autre composé qui peut notamment avoir comme effet d'améliorer la productivité et/ou la sélectivité de la réaction d'oxydation en acide adipique, comme par exemple améliorer la solubilisation de l'oxygène.

Comme exemple de tels composés, on peut citer, en particulier, les nitriles, les acides carboxyliques, plus particulièrement les acides lipophiles, les composés halogénés, plus avantageusement les composés fluorés ainsi que des précurseurs de ces composés. Comme composés plus particulièrement convenables, on peut citer les nitriles comme l'acétonitrile, le benzonitrile, des dérivés halogénés comme le dichlorométhane, les composés fluorés comme :
- Hydrocarbures aliphatiques fluorés ou perfluorés cycliques ou acycliques, hydrocarbures fluorés aromatiques tels le perfluorotoluène, perfluorométhylcyclohexane, perfluorohexane, perfluoroheptane, perfluorooctane, perfluorononane, perfluorodécaline, perfluorométhyldécaline, α,α,α-trifluorotoluène, 1,3-bis(méthyl trifluoro)benzène.
- Esters perfluorés ou fluorés tels que perfluorooctanoates d'alkyle, perfluoronanoates d'alkyle
- Cétones fluorés ou perfluorés telles que acétone perfluorée
- Alcools fluorés ou perfluorés tels que hexanol, octanol, nonanol, décanol perfluorés, t-butanol perfluoré, isopropanol perfluoré, hexafluoro-1,1,1,3,3,3-propanol-2
- Nitriles fluorés ou perfluorés tels que acétonitrile perfluoré
- Acides fluorés ou perfluorés tels que acides trifluorométhyl benzoïque, acide pentafluorobenzoique, acide hexanoique, heptanoique, octanoique, nonanoique perfluorés, acide adipique perfluoré
- Halogénures fluorés ou perfluorés tels que iodo octane perfluoré, bromooctane perfluoré
- Amines fluorées ou perfluorées tels que tripropylamine perfluorée, tributylamine perfluorée, tripentylamine perfluorée
- Acides carboxyliques comme les acides valérique, glutarique, succinique,les dérivés de l'acide aminocaproïque, les acides à caractère lipophile comme le tertiobutyle de l'acide benzoïque.

Comme acides carboxyliques lipophiles convenables pour l'invention on peut citer les acides hexanoïque, heptanoïque, octanoïque, éthyl-2 hexanoïque, nonanoïque, décanoïque, undécanoïque, dodécanoïque, stéarique (octadécanoïque) et leurs dérivés perméthylés (substitution totale des hydrogènes des groupes méthylènes par le groupe méthyle), l'acide 2-octadécylsuccinique, 2,5-ditertiobutyl benzoïque, 4-tertiobutylbenzoïque, 4-octylbenzoïque, l'hydrogénoorthophtalate de tertiobutyle, les acides naphténiques ou anthracéniques substitués par des groupements alkyles, de préférence de type tertiobutyle, les dérivés substitués des acides phtaliques, les diacides gras comme le dimère d'acide gras. On peut également citer des acides appartenant aux familles précédentes et porteurs de différents substituants électrodonneurs (groupements avec hétéroatome du type O ou N) ou électroaccepteurs (halogènes, sulfonimides, groupements nitro, sulfonanato ou analogue)

Elle peut également être mise en oeuvre en présence d'eau introduite dès le stade initial du procédé ou en contrôlant la quantité d'eau dans le milieu réactionnel soit par addition ou élimination.

Comme indiqué ci-dessus, le mélange réactionnel issu de l'oxydation est soumis à différentes opérations de séparation de certains de ses constituants pour, par exemple, permettre leur recyclage au niveau de l'oxydation et la récupération des acides produits.

Selon une première variante du procédé, on peut soumettre tout d'abord le mélange réactionnel brut à un refroidissement à une température de 16°C à 30°C par exemple, ce qui occasionne la cristallisation d'au moins une partie de l'acide formé. On obtient ainsi un milieu comprenant une phase solide constituée essentiellement d'acides, au moins une phase liquide organique contenant essentiellement le composé à oxyder n'ayant pas réagi, éventuellement le solvant et les intermédiaires d'oxydation ou autres produits résultat de l'oxydation, et une phase liquide aqueuse contenant essentiellement des sous produits acides de l'oxydation et l'eau formée. Le catalyseur peut se trouver dans une des phases organiques s'il est soluble dans ladite phase, ou dans la phase aqueuse inférieure.

Après filtration ou centrifugation du solide, on procède s'il y a lieu à la séparation par décantation des phases liquides organiques et aqueuse constituant le filtrat ou le centrifugat : la ou les phases organiques peuvent être recyclées dans une nouvelle réaction d'oxydation.

Il peut être avantageux de procéder, préalablement à l'opération de cristallisation de l'acide, à une concentration du mélange réactionnel.

Selon une deuxième variante du procédé, on peut soutirer à chaud le mélange réactionnel brut final, par exemple à une température pouvant atteindre 75°C. Le mélange réactionnel décante alors en au moins deux phases liquides : une ou plusieurs phases organiques contenant essentiellement l'hydrocarbure n'ayant pas réagi, le solvant, les intermédiaires d'oxydation et une phase liquide aqueuse contenant essentiellement les acides formés et l'eau formée. Selon la solubilité et la nature du catalyseur celui-ci peut être présent dans la ou les phases organiques, récupéré par séparation solide/liquide avant précipitation ou cristallisation de l'acide formé dans le cas d'une catalyse hétérogène ou s'il est soluble dans la phase aqueuse, extrait par extraction liquide/liquide, sur résine ou électrodialyse.

Comme dans la première variante, on procède à la séparation des phases liquides la ou les phases organiques peuvent être recyclées dans une nouvelle réaction d'oxydation.

Dans ces exemples de mode de réalisation de l'invention, de l'eau peut être ajouté au milieu réactionnel pour obtenir une meilleure dissolution des sous produits acides de l'oxydation et une meilleure récupération de l'acide formé.

La récupération de l'acide est généralement réalisée par précipitation lors du refroidissement du milieu réactionnel. L'acide ainsi récupéré peut être purifié selon des techniques habituelles et décrites dans de nombreux brevets. On peut citer, à titre d'exemple, les brevets français n° 2749299 et 2749300.

Si la phase liquide non organique ou aqueuse contient le catalyseur celui-ci est extrait soit avant la cristallisation de l'acide formé par précipitation ou extraction selon des procédés connus comme l'extraction liquide - liquide, l'électrodialyse, traitement sur résines échangeuses d'ions par exemple, soit après cristallisation de l'acide formé par des techniques d'extraction décrites ci-dessus ou analogues.

L'invention a également pour objet un procédé de fabrication d'acides carboxyliques consistant dans une première étape à oxyder un hydrocarbure en hydroperoxyde par de l'oxygène ou un gaz contenant de l'oxygène. Le milieu obtenu, après éventuellement concentration par évaporation d'une partie de l'hydrocarbure n'ayant pas réagi, est soumis à une seconde étape d'oxydation de l'hydroperoxyde en acides carboxyliques conformément au procédé de l'invention décrit ci-dessus.

Selon des modes de réalisation préférés de l'invention le milieu réactionnel issu de la première étape d'oxydation est soumis à différents traitements de séparation et élimination de sous-produits pour purifier l'hydroperoxyde. Ces traitements peuvent comprendre un lavage du milieu d'oxydation par de l'eau ou une solution légèrement basique.

D'autres avantages et détails de invention apparaîtront plus clairement au vu des exemples donnés ci-dessous uniquement à titre indicatif et d'illustration.

### Exemple 1

Dans un autoclave de 30 mL en Hastelloy C22, on charge 2,9 mg d'acétylacétonate de Mn(III) et 4,51 g d'une solution d'hydroperoxyde de cyclohexyle purifiée provenant de l'oxydation du cyclohexane, à la composition suivante exprimée en % massique:
- Hydroperoxyde de cyclohexyle: 10,59%
- Cyclohexanone: 2,06 %

L'autoclave est immédiatement pressurisé à 100 bar d'air à température ambiante et placé dans un four. Le mélange est chauffé à 130°C, sous agitation par secousses.

Après 180 minutes de réaction l'autoclave est refroidi puis dégazé. La masse réactionnelle recueillie est analysée par chromatographie en phase gazeuse (CPG).

Les résultats suivants ont été obtenus :
TT (hydroperoxyde de cyclohexyle) = 95%
TT (cyclohexane) = 2,4 %
Acide adipique : 42 mg
Par TT, on entend le taux de transformation du produit calculé par le rapport entre la différence entre le nombre de molécules initiales et le nombre de molécules finales par rapport au nombre de molécules initiales.

### Exemple 2

Dans un autoclave de 30 mL en Hastelloy C22, on charge 9,4 mg d'acétylacétonate de Mn(III) et 4,51 g d'une solution d'hydroperoxyde de cyclohexyle provenant de l'oxydation du cyclohexane, à la composition suivante exprimée en % massique pour les principaux composants :
- hydroperoxyde de cyclohexyle (HPOCH) 10,76 %
- cyclohexanol / cyclohexanone 2,58 %
- cyclohexane 85,61 %
- acides carboxyliques 0,13 %

L'autoclave est immédiatement pressurisé à 100 bar d'air à température ambiante et placé dans un four. Le mélange est chauffé à 130°C, sous agitation par secousses.

Après 180 minutes de réaction l'autoclave est refroidi puis dégazé. La masse réactionnelle recueillie est analysée par chromatographie en phase gazeuse (CPG).

Les résultats suivants ont été obtenus :
TT (hydroperoxyde de cyclohexyle) = 99,8 %
TT (cyclohexane) = 0,02 %
Acide adipique : 489 mg

### Exemple 3

Dans un autoclave de 30 mL en Hastelloy C22, on charge 7,6 mg de ScCl₃, 6 H₂O et 4,56 g d'une solution d'hydroperoxyde de cyclohexyle provenant de l'oxydation du cyclohexane, à la composition suivante exprimée en % massique pour les principaux composants :
- hydroperoxyde de cyclohexyle (HPOCH) 10,93 %
- cyclohexanol / cyclohexanone 2,29 %
- cyclohexane 85,67 %
- acides carboxyliques 0,19 %

L'autoclave est immédiatement pressurisé à 100 bar d'air à température ambiante et placé dans un four. Le mélange est chauffé à 130°C, sous agitation par secousses.

Après 180 minutes de réaction l'autoclave est refroidi puis dégazé. La masse réactionnelle recueillie est analysée par chromatographie en phase gazeuse (CPG).

Les résultats suivants ont été obtenus :
TT (hydroperoxyde de cyclohexyle) = 86,8 %
TT (cyclohexane) = 0 %
Acide adipique : 105 mg

### Exemple 4

Dans un autoclave de 30 mL en Hastelloy C22, on charge 0,4 mg de chlorure de cobalt(II) tetrahydrate et 4,50 g d'une solution d'hydroperoxyde de cyclohexyle provenant de l'oxydation du cyclohexane, à la composition suivante exprimée en % massique pour les principaux composants :
- hydroperoxyde de cyclohexyle (HPOCH) 10,93 %
- cyclohexanol / cyclohexanone 2,29 %
- cyclohexane 85,67 %
- acides carboxyliques 0,19 %

L'autoclave est immédiatement pressurisé à 100 bar d'air à température ambiante et placé dans un four. Le mélange est chauffé à 130°C, sous agitation par secousses.

Après 180 minutes de réaction l'autoclave est refroidi puis dégazé. La masse réactionnelle recueillie est analysée par chromatographie en phase gazeuse (CPG).

Les résultats suivants ont été obtenus :
TT (hydroperoxyde de cyclohexyle) = 93,3 %
TT (cyclohexane) = 1,3 %
Acide adipique : 98 mg

### Exemple 5

Dans un autoclave de 30 mL en Hastelloy C22, on charge 3 mg d'acétylacétonate de Mn(III), 142 mg d'acide valérique et 4,57 g d'une solution d'hydroperoxyde de cyclohexyle provenant de l'oxydation du cyclohexane lavée à l'eau, avec la composition suivante exprimée en % massique pour les principaux composants :
- hydroperoxyde de cyclohexyle (HPOCH) 9,85 %
- cyclohexanol / cyclohexanone 5,35 %
- cyclohexane 84,80 %

L'autoclave est immédiatement pressurisé à 100 bar d'air à température ambiante et placé dans un four. Le mélange est chauffé à 130°C, sous agitation par secousses.

Après 180 minutes de réaction l'autoclave est refroidi puis dégazé. La masse réactionnelle recueillie est analysée par chromatographie en phase gazeuse (CPG).

Les résultats suivants ont été obtenus :
TT (hydroperoxyde de cyclohexyle) = 93,5 %
TT (cyclohexane) = 0,6 %
Acide adipique : 527 mg

### Exemple 6

Dans un autoclave de 30 mL en Hastelloy C22, on charge 3 mg d'acétylacétonate de Mn(III); 455 mg d'acide para-tert-butylbenzoique et 4,5 g d'une solution d'hydroperoxyde de cyclohexyle provenant de l'oxydation du cyclohexane, à la composition suivante exprimée en % massique pour les principaux composants :
- hydroperoxyde de cyclohexyle (HPOCH) 10,76 %
- cyclohexanol / cyclohexanone 2,58 %
- cyclohexane 85,61 %
- acides carboxyliques 0,13 %

L'autoclave est immédiatement pressurisé à 100 bar d'air à température ambiante et placé dans un four. Le mélange est chauffé à 130°C, sous agitation par secousses.

Après 180 minutes de réaction l'autoclave est refroidi puis dégazé. La masse réactionnelle recueillie est analysée par chromatographie en phase gazeuse (CPG).

Les résultats suivants ont été obtenus :
TT (hydroperoxyde de cyclohexyle) = 95,4 %
TT (cyclohexane) = 3,2 %
Acide adipique : 540 mg

### Exemple 7

Dans un autoclave de 180 mL en titane, on charge 32 mg d'acétylacétonate de Mn(III) et 41,4 g d'une solution d'hydroperoxyde de cyclohexyle provenant de l'oxydation du cyclohexane, à la composition suivante exprimée en % massique pour les principaux composants :
- hydroperoxyde de cyclohexyle (HPOCH) 10,76 %
- cyclohexanol / cyclohexanone 2,58 %
- cyclohexane 85,61 %
- acides carboxyliques 0,13 %

L'autoclave est immédiatement pressurisé à 75 bar d'air à température ambiante. Le réacteur est ensuite chauffé à 130°C puis connecté à une réserve d'oxygène assurant pendant le temps de la réaction une pression partielle en oxygène de 20 bar pour une pression totale de l'autoclave de 100 bar. L'ensemble est soumis pendant toute la durée de la réaction à une agitation de 1000 tours par minute.

Après 80 minutes de réaction l'autoclave est refroidi puis dégazé. La masse réactionnelle recueillie est analysée par chromatographie en phase gazeuse (CPG).

Les résultats suivants ont été obtenus :
TT (hydroperoxyde de cyclohexyle) = 99,9 %
TT (cyclohexane) = 6,2 %
Acide adipique : 7,34 g
Après refroidissement de la solution et filtration on obtient un solide blanc contenant majoritairement de l'acide adipique. La productivité associée à cet essai est de l'ordre de 100 gramme d'acide adipique produit par litre de milieu réactionnel et par heure.

### Exemple 8

Dans un autoclave de 180 mL en titane, on charge 73 mg d'acétylacétonate de Mn(III) et 42,6 g d'une solution d'hydroperoxyde de cyclohexyle provenant de l'oxydation du cyclohexane, à la composition suivante exprimée en % massique pour les principaux composants :
- hydroperoxyde de cyclohexyle (HPOCH) 10,76 %
- cyclohexanol / cyclohexanone 2,58 %
- cyclohexane 85,61 %
- acides carboxyliques 0,13 %

L'autoclave est immédiatement pressurisé à 75 bar d'air à température ambiante. Le réacteur est ensuite chauffé à 130°C puis connecté à une réserve d'oxygène assurant pendant le temps de la réaction une pression partielle en oxygène de 20 bar pour une pression totale de l'autoclave de 100 bar. L'ensemble est soumis pendant toute la durée de la réaction à une agitation de 1000 tours par minute.

Après 55 minutes de réaction l'autoclave est refroidi puis dégazé. La masse réactionnelle recueillie est analysée par chromatographie en phase gazeuse (CPG).

Les résultats suivants ont été obtenus :
TT (hydroperoxyde de cyclohexyle) = 100 %
TT (cyclohexane) = 6,3 %
Acide adipique : 7,32 g
Après refroidissement de la solution et filtration on obtient un solide blanc contenant majoritairement de l'acide adipique. La productivité associée à cet essai est de l'ordre de 130 gramme d'acide adipique produit par litre de milieu réactionnel et par heure.

## Revendications

1. Procédé de fabrication d'acides carboxyliques, **caractérisé en ce qu'**il consiste à faire réagir un hydroperoxyde organique obtenu par oxydation d'alcanes, cycloalcanes ou hydrocarbures alkyl-aromatiques avec un agent d'oxydation comprenant de l'oxygène moléculaire et en présence d'un catalyseur d'oxydation, après élimination des sous-produits autres que le composé hydroperoxyde de l'oxydation des alcanes, cycloalcanes ou hydrocarbures alkyl-aromatiques.

2. Procédé selon la revendication 1, **caractérisé en ce que** l'hydroperoxyde organique est choisi dans le groupe comprenant les hydroperoxydes primaires ou secondaires dérivant des alcanes, des cycloalcanes, des hydrocarbures alkyl-aromatiques dont le cycle aromatique comporte éventuellement un ou plusieurs substituants tels que notamment groupe alkyle ou atome d'halogène plus particulièrement atome de chlore, des alcènes et des cycloalcènes ayant de 3 à 20 atomes de carbone.

3. Procédé selon l'une des revendications 1 à 2, **caractérisé en ce que** hydroperoxyde organique est choisi dans le groupe comprenant l'hydroperoxyde de cyclohexyle, l'hydroperoxyde de cyclododécyle, l'hydroperoxyde de la tétraline, l'hydroperoxyde d'éthylbenzène, l'hydroperoxyde du pinane.

4. Procédé selon l'une des revendications précédentes, **caractérisé en ce que** le milieu réactionnel issu de l'oxydation d'un hydrocarbure est soumis à un lavage par l'eau avant d'être mis en réaction avec un agent d'oxydation.

5. Procédé selon l'une des revendications précédentes, **caractérisé en ce que** le catalyseur est soluble dans le milieu liquide aux conditions de mise en oeuvre de la réaction d'oxydation.

6. Procédé selon l'une des revendications 1 à 4, **caractérisé en ce que** le catalyseur est insoluble dans le milieu liquide aux conditions de mise en oeuvre de la réaction d'oxydation.

7. Procédé selon la revendication 6, **caractérisé en ce que** le catalyseur est un catalyseur supporté comprenant un support minéral ou polymérique.

8. Procédé selon l'une des revendications précédentes **caractérisé en ce que** la réaction d'oxydation est réalisée en présence d'un solvant.

9. Procédé selon la revendication 8, **caractérisé en ce que** le solvant est choisi dans le groupe comprenant les alcanes, les cycloalcanes, les hydrocarbures aromatiques, les hydrocarbures halogénés, les alcools, les cétones, les éthers et les mélanges de ces solvants

10. Procédé selon la revendication 9, **caractérisé en ce que** le solvant est choisi dans le groupe comprenant l'hexane, l'heptane, l'isooctane, le cyclohexane, le cyclooctane, le benzène, le toluène et le xylène.

11. Procédé selon la revendication 10, **caractérisé en ce que** le solvant est l'hydrocarbure correspondant à l'hydroperoxyde à oxyder.

12. Procédé selon l'une des revendications précédentes, **caractérisé en ce que** le milieu liquide, après oxydation, est décanté, en au moins une phase organique formées par l'hydroperoxyde non oxydé, le solvant, et une phase aqueuse ou solide, lesdites phases organiques étant recyclées dans une nouvelle oxydation, l'acide produit étant extrait de la phase aqueuse.

13. Procédé selon la revendication 12, **caractérisé en ce que** l'acide est extrait de la phase aqueuse par cristallisation.

14. Procédé selon l'une des revendications précédentes, **caractérisé en ce que** le catalyseur est recyclé avec la ou les phases organiques.

15. Procédé selon l'une des revendications précédentes, **caractérisé en ce que** le catalyseur est séparé du milieu liquide par décantation ou séparation solide/liquide.

16. Procédé selon l'une des revendications 10 ou 11, **caractérisé en ce que** le catalyseur soluble dans la phase aqueuse est extrait par extraction liquidel/iquide, séparation sur résines, ou par électrodialyse.

17. Procédé selon l'une des revendications précédentes, **caractérisé en ce que** le catalyseur comprend du manganèse comme élément catalytiquement actif.

18. Procédé de fabrication d'acides carboxyliques selon l'une des revendications précédentes, **caractérisé en ce que** l'hydroperoxyde organique est obtenu par oxydation d'alcanes, cycloalcanes ou hydrocarbures alkyl-aromatiques par de l'oxygène ou un gaz contenant de l'oxygène, le milieu réactionnel d'oxydation étant concentré en hydroperoxyde par extraction d'au moins une partie des alcanes, cycloalcanes ou hydrocarbures alkyl-aromatiques non oxydés, préalablement à l'oxydation des hydroperoxydes en acides carboxyliques.

## Claims

1. Process for producing carboxylic acids, **characterized in that** it consists in reacting an organic hydroperoxide obtained by oxidation of alkanes, cycloalkanes or alkylaromatic hydrocarbons with an oxidizing agent comprising molecular oxygen and in the presence of an oxidation catalyst, after elimination of the by-products other than the hydroperoxide compound from the oxidation of alkanes, cycloalkanes or alkylaromatic hydrocarbons.

2. Process according to Claim 1, **characterized in that** the organic hydroperoxide is chosen from the group comprising primary or secondary hydroperoxides deriving from alkanes, cycloalkanes, alkylaromatic hydrocarbons, the aromatic ring of which optionally comprises one or more substituents such as, in particular, an alkyl group or a halogen atom, more particularly a chlorine atom, alkenes and cycloalkenes containing from 3 to 20 carbon atoms.

3. Process according to either of Claims 1 and 2, **characterized in that** the organic hydroperoxide is chosen from the group comprising cyclohexyl hydroperoxide, cyclododecyl hydroperoxide, tetraline hydroperoxide, ethylbenzene hydroperoxide and pinane hydroperoxide.

4. Process according to one of the preceding claims, **characterized in that** the reaction medium derived from the oxidation of a hydrocarbon is subjected to washing with water before being reacted with an oxidizing agent.

5. Process according to one of the preceding claims, **characterized in that** the catalyst is soluble in the liquid medium under the conditions under which the oxidation reaction is carried out.

6. Process according to either of Claims 1 and 4, **characterized in that** the catalyst is insoluble in the liquid medium under the conditions under which the oxidation reaction is carried out.

7. Process according to Claim 6, **characterized in that** the catalyst is a supported catalyst comprising an inorganic or polymeric support.

8. Process according to one of the preceding claims, **characterized in that** the oxidation reaction is carried out in the presence of a solvent.

9. Process according to Claim 8, **characterized in that** the solvent is chosen from the group comprising alkanes, cycloalkanes, aromatic hydrocarbons, halogenated hydrocarbons, alcohols, ketones and ethers, and mixtures of these solvents.

10. Process according to Claim 9, **characterized in that** the solvent is chosen from the group comprising hexane, heptane, isooctane, cyclohexane, cyclooctane, benzene, toluene and xylene.

11. Process according to Claim 10, **characterized in that** the solvent is the hydrocarbon corresponding to the hydroperoxide to be oxidized.

12. Process according to one of the preceding claims, **characterized in that** the liquid medium, after oxidation, is separated by settling into at least one organic phase formed by the nonoxidized hydroperoxide, the solvent, and an aqueous or solid phase, said organic phases being recycled in a further oxidation, the acid produced being extracted from the aqueous phase.

13. Process according to Claim 12, **characterized in that** the acid is extracted from the aqueous phase by crystallization.

14. Process according to one of the preceding claims, **characterized in that** the catalyst is recycled with the organic phase(s).

15. Process according to one of the preceding claims, **characterized in that** the catalyst is separated from the liquid medium by settling or by solid/liquid separation.

16. Process according to either of Claims 10 and 11, **characterized in that** the catalyst soluble in the aqueous phase is extracted by liquid/liquid extraction, separation on resins, or by electrodialysis.

17. Process according to one of the preceding claims, **characterized in that** the catalyst comprises manganese as catalytically active element.

18. Process for producing carboxylic acids according to one of the preceding claims, **characterized in that** the organic hydroperoxide is obtained by oxidation of alkanes, cycloalkanes or alkylaromatic hydrocarbons with oxygen or a gas containing oxygen, the oxidation reaction medium being concentrated with respect to hydroperoxide by extraction of at least part of the nonoxidized alkanes, cycloalkanes or alkylaromatic hydrocarbons, prior to the oxidation of the hydroperoxides to carboxylic acids.

## Patentansprüche

1. Verfahren zur Herstellung von Carbonsäuren, **dadurch gekennzeichnet, dass** man ein organisches Hydroperoxid, das durch Oxidation von Alkanen. Cycloalkanen oder alkylaromatischen Kohlenwasserstoffen erhalten wurde, mit einem Oxidationsmittel, welches molekularen Sauerstoff einschließt, und in Gegenwart eines Oxidationskatalysators nach Abtrennung der Nebenprodukte, die anders sind als die Hydroperoxidverbindung der Oxidation von Alkanen, Cycloalkanen oder alkylaromatischen Kohlenwasserstoffen, reagieren lässt.

2. Verfahren nach Anspruch 1, **dadurch gekennzeichnet, dass** das organische Hydroperoxid aus der Gruppe ausgewählt ist, die die primären oder sekundären Hydroperoxide einschließt, die sich von Alkanen, Cycloalkanen, alkylaromatischen Kohlenwasserstoffen, deren aromatischer Cyclus gegebenenfalls einen oder mehrere Substituenten einschließt, wie insbesondere eine Alkylgruppe oder ein Halogenatom, ganz besonders ein Chloratom, Alkenen und Cycloalkenen mit 3 bis 20 Kohlenstoffatomen ableiten.

3. Verfahren nach einem der Ansprüche 1 bis 2, **dadurch gekennzeichnet, dass** das organische Hydroperoxid aus der Gruppe ausgewählt ist, die Cyclohexylhydroperoxid, Cyclododecylhydroperoxid. Tetralinhydroperoxid. Ethylbenzolhydroperoxid, Pinanhydroperoxid einschließt.

4. Verfahren nach einem der vorhergehenden Ansprüche, **dadurch gekennzeichnet, dass** das Reaktionsmedium, das aus der Oxidation eines Kohlenwasserstoffs hervorgeht, einer Wäsche mit Wasser unterzogen wird, bevor es mit einem Oxidationsmittel zur Reaktion gebracht wird.

5. Verfahren nach einem der vorhergehenden Ansprüche, **dadurch gekennzeichnet, dass** der Katalysator in dem flüssigen Medium unter den Bedingungen der Durchführung der Oxidationsreaktion löslich ist.

6. Verfahren nach einem der Ansprüche 1 bis 4. **dadurch gekennzeichnet, dass** der Katalysator in dem flüssigen Medium unter den Bedingungen der Durchführung der Oxidationsreaktion unlöslich ist.

7. Verfahren nach Anspruch 6. **dadurch gekennzeichnet, dass** der Katalysator ein geträgerter Katalysator ist, der einen mineralischen oder polymeren Träger einschließt.

8. Verfahren nach einem der vorhergehenden Ansprüche, **dadurch gekennzeichnet, dass** die Oxidationsreaktion in Gegenwart eines Lösungsmittels durchgeführt wird.

9. Verfahren nach Anspruch 8, **dadurch gekennzeichnet, dass** das Lösungsmittel aus der Gruppe ausgewählt ist, die Alkane, Cycloalkane, aromatische Kohlenwasserstoffe, halogenierte Kohlenwasserstoffe, Alkohole, Ketone, Ether und Gemische dieser Lösungsmittel einschließt.

10. Verfahren nach Anspruch 9. **dadurch gekennzeichnet, dass** das Lösungsmittel aus der Gruppe ausgewählt ist, die Hexan, Heptan, Isooctan, Cyclohexan, Cyclooctan, Benzol, Toluol und Xylol einschließt.

11. Verfahren nach Anspruch 10. **dadurch gekennzeichnet, dass** das Lösungsmittel der Kohlenwasserstoff ist, der dem zu oxidierenden Hydroperoxid entspricht.

12. Verfahren nach einem der vorhergehenden Ansprüche, **dadurch gekennzeichnet, dass** das flüssige Medium, nach Oxidation, in mindestens eine organische Phase, die durch nicht oxidiertes Hydroperoxid gebildet wird, das Lösungsmittel und eine wässrige oder feste Phase dekantiert wird, wobei die organischen Phasen in eine neue Oxidation recycelt werden, wobei die produzierte Säure aus der wässrigen Phase extrahiert wird.

13. Verfahren nach Anspruch 12, **dadurch gekennzeichnet, dass** die Säure aus der wässrigen Phase durch Kristallisation extrahiert wird.

14. Verfahren nach einem der vorhergehenden Ansprüche, **dadurch gekennzeichnet, dass** der Katalysator mit der oder den organischen Phasen recycelt wird.

15. Verfahren nach einem der vorhergehenden Ansprüche, **dadurch gekennzeichnet, dass** der Katalysator von dem flüssigen Medium durch Dekantieren oder Fest/Flüssig-Trennung abgetrennt wird.

16. Verfahren nach einem der Ansprüche 10 oder 11, **dadurch gekennzeichnet, dass** der in der wässrigen Phase lösliche Katalysator durch Flüssig/Flüssig-Extraktion, Trennung über Harze oder durch Elektrodialyse extrahiert wird.

17. Verfahren nach einem der vorhergehenden Ansprüche, **dadurch gekennzeichnet, dass** der Katalysator Mangan als katalytisch aktives Element einschließt.

18. Verfahren zur Herstellung von Carbonsäuren nach einem der vorhergehenden Ansprüche, **dadurch gekennzeichnet, dass** das organische Hydroperoxid durch Oxidation von Alkanen, Cycloalkanen oder alkylaromatischen Kohlenwasserstoffen durch Sauerstoff oder ein Gas erhalten wird, das Sauerstoff enthält, wobei das Oxidationsreaktionsmedium vor der Oxidation der Hydroperoxide zu Carbonsäuren durch Extraktion von mindestens einem Teil der Alkane, Cycloalkane oder alkylaromatischen nicht oxidierten Kohlenwasserstoffe, an Hydroperoxid konzentriert wird.
